(19) Europäisches Patentamt
European Patent Office
Office européen des brevets

(11) Veröffentlichungsnummer: **0 101 902**
**A1**

# (12) EUROPÄISCHE PATENTANMELDUNG

(21) Anmeldenummer: **83107180.8**

(22) Anmeldetag: **22.07.83**

(51) Int. Cl.³: **C 07 D 239/92**
C 07 D 239/96, A 01 N 47/18

(30) Priorität: **03.08.82 DE 3228871**

(43) Veröffentlichungstag der Anmeldung:
**07.03.84 Patentblatt 84/10**

(84) Benannte Vertragsstaaten:
**BE CH DE FR GB LI**

(71) Anmelder: **BAYER AG**
**Konzernverwaltung RP Patentabteilung**
**D-5090 Leverkusen 1 Bayerwerk(DE)**

(72) Erfinder: **Maurer, Fritz, Dr.**
**Roeberstrasse 8**
**D-5600 Wuppertal 1(DE)**

(72) Erfinder: **Brandes, Wilhelm, Dr.**
**Eichendorffstrasse 3**
**D-5653 Leichlingen(DE)**

(72) Erfinder: **Kuck, Karl-Heinz, Dr.**
**Herrstrasse 24**
**D-4018 Langenfeld(DE)**

(72) Erfinder: **Reinecke, Paul, Dr.**
**Lessingstrasse 11**
**D-5090 Leverkusen 3(DE)**

(72) Erfinder: **Scheinpflug, Hans, Dr.**
**Am Thelenhof 15**
**D-5090 Leverkusen 1(DE)**

## (54) Oxochinazolin-carbaminsäureester.

(57) Die vorliegende Erfindung betrifft neue Oxochinazolin-carbaminsäureester der Formel

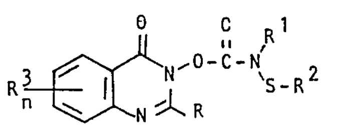

(I)

in welcher

R für Wasserstoff, Alkyl, Alkoxy, Alkoxyalkyl, Alkylthio, Alkylthioalkyl, Halogenalkyl oder für gegebenenfalls substituiertes Aryl steht,
$R^1$ für Alkyl steht,
$R^2$ für Halogenalkyl steht,
$R^3$ für Alkyl oder Halogen steht und
n für eine Zahl von 0 bis 4 steht.

Sie werden erhalten durch Umsetzung von 3-Hydroxy-4-oxo-3,4-dihydrochinazolinen der Formel

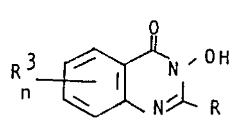

(II)

mit Carbamoylhalogeniden der Formel III

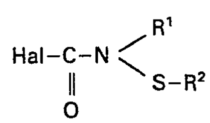

(III),

in Gegenwart eines Säurebindemittels und gegebenenfalls in Gegenwart eines Verdünnungsmittels.

Die neuen Oxochinazolin-carbaminsäureester der Formel I können als Schädlingsbekämpfungsmittel, insbesondere als Fungizide und Bakterizide, eingesetzt werden und sind den aus dem Stand der Technik bekannten Verbindungen in ihrer Wirkung überlegen.

EP 0 101 902 A1

BAYER AKTIENGESELLSCHAFT 5090 Leverkusen, Bayerwerk
Zentralbereich
Patente, Marken und Lizenzen

02.08.82
Bas/ABc
Ia

## Oxochinazolin-carbaminsäurester

Die Erfindung betrifft neue Oxochinazolin-carbaminsäureester, ein Verfahren zu ihrer Herstellung und ihre Verwendung als Schädlingsbekämpfungsmittel.

Es ist bereits bekannt geworden, daß eine Reihe von anorganischen und organischen chemischen Verbindungen, wie z.B. basisches Kupferchlorid oder Zink-ethylen-1,2-bis-(dithiocarbamat) oder N-Trichlormethylthio-tetrahydrophthalimid fungizide und bakterizide Eigenschaften aufweisen. (Vergleiche z.B. R.Wegler, "Chemie der Pflanzenschutz- und Schädlingsbekämpfungsmittel", Springer Verlag Berlin, Band 2.) Die Wirkung ist jedoch, insbesondere bei niedrigen Aufwandmengen und -konzentrationen, nicht immer voll befriedigend.

Es wurden neue Oxochinazolin-carbaminsäureester der allgemeinen Formel (I)

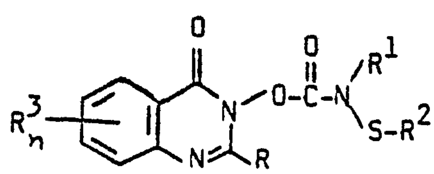

(I)

in welcher

R für Wasserstoff, Alkyl, Alkoxy, Alkoxyalkyl, Alkylthio, Alkylthioalkyl, Halogenalkyl oder gegebenenfalls substituiertes Aryl steht,

$R^1$ für Alkyl steht,

$R^2$ für Halogenalkyl steht,

$R^3$ für Alkyl oder Halogen steht und

n für eine Zahl von 0 bis 4 steht,

gefunden.

Weiterhin wurde gefunden, daß man die neuen Oxochinazolin-carbaminsäureester der Formel (I)

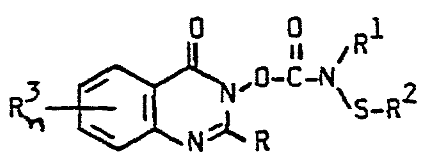

(I)

in welcher

R für Wasserstoff, Alkyl, Alkoxy, Alkoxyalkyl, Alkylthio, Alkylthioalkyl, Halogenalkyl oder gegebenenfalls substituiertes Aryl steht,

$R^1$ für Alkyl steht,

$R^2$ für Halogenalkyl steht,

$R^3$ für Alkyl oder Halogen steht und

n für eine Zahl von 0 bis 4 steht,

erhält, wenn man 3-Hydroxy-4-oxo-3,4-dihydrochinazoline der Formel (II)

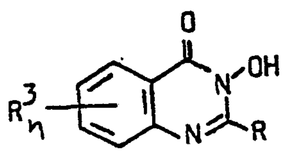

(II)

in welcher

R, $R^3$ und n die oben angegebene Bedeutung haben,

mit Carbamoylhalogeniden der Formel (III)

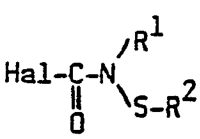

(III)

in welcher

$R^1$ und $R^2$ die oben angegebene Bedeutung haben und

Hal für Halogen steht,

in Gegenwart eines Säurebindemittels und gegebenenfalls in Gegenwart eines Verdünnungsmittels umsetzt.

Die neuen Oxochinazolin-carbaminsäureester der Formel (I) weisen starke fungizide und bakterizide Eigenschaften auf. Dabei zeigen die erfindungsgemäßen Verbindungen der Formel (I) überraschenderweise eine bessere fungizide und bakterizide Wirksamkeit, als die nach dem Stand der Technik bekannten fungiziden Stoffe:

Zink-ethylen-1,2-bis-(dithiocarbamat) oder N-Trichlormethylthiotetrahydrophthalimid bzw. als bekannter bakterizider Stoff basisches Kupferchlorid. Die erfindungsgemäßen Stoffe stellen somit eine Bereicherung der Technik dar.

Die erfindungsgemäßen Oxochinazolin-carbaminsäureester sind durch die Formel (I) allgemein definiert. In dieser Formel stehen vorzugsweise

R für Wasserstoff, für geradkettiges oder verzweigtes Alkyl, Alkoxy, Alkoxyalkyl, Alkylthio oder Alkylthioalkyl mit jeweils bis zu 6 Kohlenstoffatomen je Alkylrest, für Halogenalkyl mit bis zu 3 Kohlenstoffatomen und bis zu 7 gleichen oder verschiedenen Halogenatomen, insbesondere Fluor, Chlor oder Brom, sowie für gegebenenfalls einfach oder mehrfach gleich oder verschieden substituiertes Aryl mit 6 bis 10 Kohlenstoffatomen, wobei als Substituenten in Frage kommen: Alkyl mit bis zu 4 Kohlenstoffatomen, Halogenalkyl mit bis zu 3 Kohlenstoffatomen und bis zu 5 Halogenatomen und Halogen, insbesondere Fluor, Chlor oder Brom,

$R^1$ für geradkettiges oder verzweigtes Alkyl mit bis zu 6 Kohlenstoffatomen,

$R^2$ für Halogenalkyl mit bis zu 3 Kohlenstoffatomen und bis zu 7 gleichen oder verschiedenen Halogenatomen, insbesondere Fluor, Chlor oder Brom,

$R^3$ für geradkettiges oder verzweigtes Alkyl mit bis zu 4 Kohlenstoffatomen oder für Halogen, insbesondere Fluor, Chlor oder Brom und

n für eine Zahl von 0 bis 3.

Besonders bevorzugt sind diejenigen Verbindungen der Formel (I), in denen

R für Wasserstoff, für geradkettiges oder verzweigtes Alkyl mit 1 bis 4 Kohlenstoffatomen, wie Methyl, Ethyl, iso-Propyl, n-Propyl, n-Butyl, iso-Butyl, sec.-Butyl oder tert.-Butyl, für Alkoxy mit 1 bis 4 Kohlenstoffatomen, wie Methoxy, Ethoxy, n-Propoxy, iso-Propoxy, n-Butoxy, für Alkoxyalkyl mit 1 bis 4 Kohlenstoffatomen je Alkylrest, wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 2-Methoxyethyl,

2-Ethoxy-ethyl, 2-n-Propoxy-ethyl, 2-iso-Propoxy-ethyl, 2-n-Butoxy-ethyl, 2-iso-Butoxy-ethyl, 2-tert.-Butoxy-ethyl, 2-sec.-Butoxy-ethyl, 2-Methoxy-n-propyl, 2-Ethoxyn-propyl, 2-n-Propoxy-n-propyl, 2-iso-Propoxy-n-propyl oder 4-Methoxy-n-butyl, für Alkylthio mit 1 bis 4 Kohlenstoffatomen, wie Methylthio, Ethylthio, n-Propylthio, iso-Propylthio, n-Butylthio, iso-Butylthio, sec.-Butylthio oder tert. -Butylthio, für Alkylthioalkyl mit 1 bis 4 Kohlenstoffatomen je Alkylrest, wie Methylthiomethyl, Ethylthiomethyl, n-Propylthiomethyl, iso-Propylthiomethyl, n-Butylthiomethyl, iso-Butylthiomethyl, sec.-Butylthiomethyl, tert.-Butylthiomethyl, 2-Methylthio-ethyl, 2-Ethylthio-ethyl, 2-n-Propylthio-ethyl, 2-iso-Propylthio-ethyl, 2-n-Butylthio-ethyl, 2-iso-Butylthio-ethyl, 2-tert.-Butylthio-ethyl, 3-Methylthio-n-propyl, 3-Ethylthio-n-propyl, 3-n-Propylthio-n-propyl, 3-iso-Propylthio-n-propyl, 3-n-Butylthio-n-propyl, 3-tert.-Butylthio-n-propyl, 4-Methylthio-n-butyl oder 4-Ethylthio-n-butyl, für Halogenalkyl mit 1 bis 2 Kohlenstoffatomen und 1 bis 5 gleichen oder verschiedenen Halogenatomen, insbesondere Fluor, Chlor oder Brom, wie z.B. Trichlormethyl, Trifluormethyl, Dichlorfluormethyl, Chlordifluormethyl, Pentachlorethyl und für gegebenenfalls ein- bis dreifach, gleich oder verschieden substituiertes Phenyl steht, wobei als Substituenten besonders bevorzugt sind: Fluor, Chlor, Brom, Methyl, Ethyl, Trifluormethyl und Trichlormethyl,

$R^1$ für geradkettiges oder verzweigtes Alkyl mit 1 bis 4 Kohlenstoffatomen steht, wie Methyl, Ethyl, n-Propyl, iso-Propyl, n-Butyl, iso-Butyl, sec.-Butyl oder tert.-Butyl,

$R^2$ für Halogenalkyl mit 1 oder 2 Kohlenstoffatomen und 1 bis 5 gleichen oder verschiedenen Halogenatomen, insbesondere Fluor, Chlor oder Brom steht, wie Trichlormethyl, Trifluormethyl, Dichlorfluormethyl, Chlordifluormethyl, Pentachlorethyl,

$R^3$ für Methyl, Ethyl, Fluor, Chlor oder Brom steht und

n für die Zahl 0, 1 oder 2 steht.

Im einzelnen seien außer den bei den Herstellungsbeispielen aufgeführten Verbindungen die folgenden Verbindungen der Formel (I) genannt:

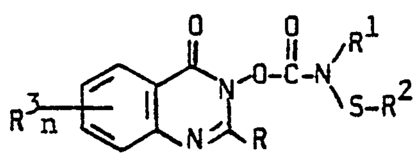

(I)

| R | $R^1$ | $R^2$ | $R^3$ | n |
|---|---|---|---|---|
| $-CH_3$ | $-CH_3$ | $-CCl_3$ | - | 0 |
| $-CH_3$ | $-C_2H_5$ | $-CCl_2F$ | - | 0 |
| $-CH_3$ | $n-C_3H_7-$ | $-CCl_2F$ | - | 0 |
| $-CH_3$ | $i-C_3H_7-$ | $-CCl_2F$ | - | 0 |
| $-CH_3$ | $n-C_4H_9-$ | $-CCl_2F$ | - | 0 |
| $-CH_3$ | $i-C_4H_9-$ | $-CCl_2F$ | - | 0 |
| $-CH_3$ | $t-C_4H_9-$ | $-CCl_2F$ | - | 0 |

| R | $R^1$ | $R^2$ | $R^3$ | n |
|---|---|---|---|---|
| $n-C_3H_7-$ | $-CH_3$ | $-CCl_2F$ | - | 0 |
| $i-C_3H_7-$ | $-CH_3$ | $-CCl_2F$ | - | 0 |
| $-OCH_3$ | $-C_2H_5$ | $-CCl_2F$ | - | 0 |
| $-OC_2H_5$ | $-CH_3$ | $-CCl_2F$ | - | 0 |
| $-CH_2Cl$ | $-CH_3$ | $-CCl_2F$ | - | 0 |
| $-CHCl_2$ | $-CH_3$ | $-CCl_2F$ | - | 0 |
| $-CF_3$ | $-CH_3$ | $-CCl_2F$ | - | 0 |
| $-CCl_3$ | $-CH_3$ | $-CCl_2F$ | - | 0 |
| $-CH_2-SCH_3$ | $-CH_3$ | $-CCl_2F$ | - | 0 |
| $-C_6H_5$ | $-CH_3$ | $-CCl_3$ | - | 0 |
| $-C_6H_5$ | $-C_2H_5$ | $-CCl_2F$ | - | 0 |
| 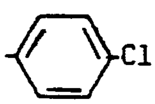 | $-CH_3$ | $-CCl_2F$ | - | 0 |
| 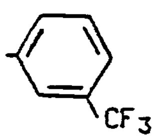 | $-CH_3$ | $-CCl_2F$ | - | 0 |
| $-CH_3$ | $-CH_3$ | $-CCl_3$ | $CH_3$ | 1 |
| $-CH_3$ | $-C_2H_5$ | $-CCl_2F$ | $CH_3$ | 1 |
| $-CH_3$ | $n-C_3H_7-$ | $-CCl_2F$ | $CH_3$ | 1 |
| $-CH_3$ | $i-C_3H_7-$ | $-CCl_2F$ | $CH_3$ | 1 |
| $-CH_3$ | $n-C_4H_9-$ | $-CCl_2F$ | $C_2H_5$ | 1 |
| $-CH_3$ | $i-C_4H_9-$ | $-CCl_2F$ | $C_2H_5$ | 1 |
| $-CH_3$ | $t-C_4H_9-$ | $-CCl_2F$ | F | 1 |

Fortsetzung

| R | $R^1$ | $R^2$ | $R^3$ | n |
|---|---|---|---|---|
| $n-C_3H_7$ | $-CH_3$ | $-CCl_2F$ | F | 1 |
| $i-C_3H_7$ | $-CH_3$ | $-CCl_2F$ | Cl | 1 |
| $-OCH_3$ | $-C_2H_5$ | $-CCl_2F$ | Cl | 1 |
| $-OC_2H_5$ | $-CH_3$ | $-CCl_2F$ | $CH_3$ | 1 |
| $-CH_2Cl$ | $-CH_3$ | $-CCl_2F$ | Br | 1 |
| $-CHCl_2$ | $-CH_3$ | $-CCl_2F$ | Br | 1 |
| $-CF_3$ | $-CH_3$ | $-CCl_2F$ | Br | 1 |
| $-CCl_3$ | $-CH_3$ | $-CCl_2F$ | $C_2H_5$ | 1 |
| $-CH_2-SCH_3$ | $-CH_3$ | $-CCl_2F$ | $C_2H_5$ | 1 |
| $-C_6H_5$ | $-CH_3$ | $-CCl_3$ | $C_2H_5$ | 1 |
| $-C_6H_5$ | $-C_2H_5$ | $-CCl_2F$ | $CH_3$ | 1 |
| 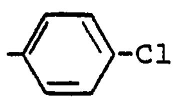 | $-CH_3$ | $-CCl_2F$ | $C_2H_5$ | 1 |
| 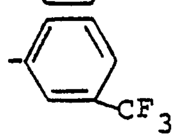 | $-CH_3$ | $-CCl_2F$ | $C_2H_5$ | 1 |

Verwendet man beispielsweise 2-Ethyl-3-hydroxy-3,4-dihydro-4-oxo-chinazolin und N-Methyl-N-dichlorfluormethylsulfenyl-carbamoylfluorid als Ausgangsstoffe, so kann der Reaktionsablauf des erfindungsgemäßen Verfahrens durch das folgende Formelschema wiedergegeben werden:

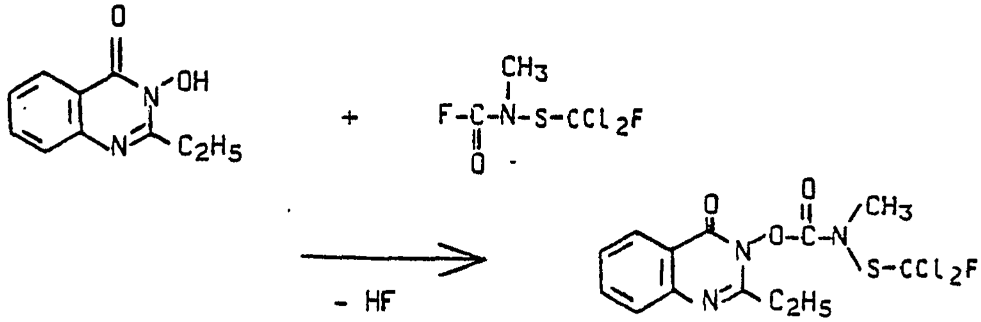

Die bei der Durchführung des erfindungsgemäßen Verfahrens als Ausgangsstoffe zu verwendenden 3-Hydroxy-4-oxo-3,4-dihydrochinazoline sind durch die Formel (II) allgemein definiert. In dieser Formel stehen R, $R^3$ und n vorzugsweise für diejenigen Reste, die bereits im Zusammenhang mit der Beschreibung der erfindungsgemäßen Stoffe der Formel (I) vorzugsweise für diese Substituenten genannt wurden.

Die 3-Hydroxy-4-oxo-3,4-dihydrochinazoline der Formel (II) sind teilweise bekannt (vgl. z.B. C.B. Schapira und S. Lamdan, J. Heterocycl. Chem. 9, 569 (1972)).

Man erhält sie beispielsweise, indem man o-Aminobenzhydroxamsäuren der Formel (IV)

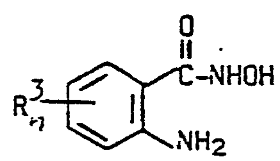

(IV)

in welcher
$R^3$ und n die oben angegebene Bedeutung haben,

mit Acylierungsmitteln wie z.B. Acylhalogeniden oder Carbonsäureorthoestern in Gegenwart eines Lösungsmittels bei Temperaturen zwischen 20°C und 150°C gegebenenfalls in Gegenwart eines Katalysators cyclisiert.

Die o-Aminobenzhydroxamsäuren der Formel (IV) sind bekannt (vergleiche z.B. A.W.Scott und B.L.Wood, J.Org. Chem. 7, 508 (1942)).

Die weiterhin als Ausgangsstoffe zu verwendenden Carbamoylhalogenide sind durch die Formel (III) allgemein definiert. In dieser Formel stehen $R^1$ und $R^2$ vorzugsweise für diejenigen Reste, die bereits im Zusammenhang mit der Beschreibung der erfindungsgemäßen Stoffe der Formel (I) vorzugsweise für diese Substituenten genannt wurden. Hal steht vorzugsweise für Fluor oder Chlor.

Die Carbamoylhalogenide der Formel (III) sind bekannt (vgl. z.B. Belg. Patent Nr. 717 705).

Für die erfindungsgemäße Umsetzung kommen als Verdünnungsmittel vorzugsweise alle inerten organischen Lösungsmittel in Frage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Ether, wie Diethylether, Diisopropylether, Dioxan und Tetrahydrofuran, Ketone, wie Aceton, Butanon, Methylisopropyl- und Methylisobutylketon, Nitrile, wie Acetonitril und Propionitril sowie die hochpolaren Lösungsmittel Dimethylsulfoxid und Hexamethylphosphorsäuretriamid.

Das erfindungsgemäße Verfahren wird in Gegenwart von Säurebindern vorgenommen. Man kann alle üblicherweise verwendbaren anorganischen und organischen Säurebinder zugeben. Hierzu gehören vorzugsweise Alkalicarbonate, wie beispielsweise Natriumcarbonat, Kaliumcarbonat und Natriumhydrogencarbonat, ferner niedere tertiäre Alkylamine, Cycloalkylamine oder Arylalkylamine, wie beispielsweise Triethylamin, N,N-Dimethyl-benzylamin, weiterhin Pyridin sowie 1,4-Diazabicyclo(2,2,2)-octan und 1,5-Diazabicyclo-(4,3,0)-non-5-en.

Die Reaktionstemperatur kann bei der Durchführung des erfindungsgemäßen Verfahrens innerhalb eines größeren Bereiches variiert werden. Im allgemeinen arbeitet man zwischen 0°C und 100°C, vorzugsweise zwischen 10°C und 80°C.

Das erfindungsgemäße Verfahren wird im allgemeinen bei Normaldruck durchgeführt.

Zur Durchführung des erfindungsgemäßen Verfahrens setzt man auf 1 Mol 3-Hydroxy-4-oxo-3,4-dihydrochinazolin der Formel (II) gewöhnlich zwischen 1 und 1,5 Mol, vorzugsweise zwischen 1 und 1,2 Mol Carbamoylhalogenid der Formel (III) ein.

Die Umsetzung wird vorzugsweise unter Verwendung eines der oben genannten Säurebinder in einem der oben angegebenen Verdünnungsmittel durchgeführt. Das Reaktionsgemisch wird mehrere Stunden bei der erforderlichen Temperatur gerührt. Die Aufarbeitung der Reaktionsmischung und die Isolierung der erfindungsgemäßen Reaktionsprodukte der Formel (I) erfolgt in allgemein üblicher Art und Weise.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe der Formel (I) mit besonders gutem Erfolg zur Bekämpfung von Botrytis-Arten wie z.B. den Erreger des Grauschimmels an Früchten (Botrytis cinerea) zur Bekämpfung von Leptosphaeria-Arten, wie z.B. den Erreger der Braunfleckenkrankheit an Weizen (Leptosphaeria nodorum) sowie zur Bekämpfung von Xanthomonas-Arten, wie z.B. den Erreger der Streifenkrankheit an Reis (Xanthomonas oryzae), ebenso wie zur Bekämpfung von Getreiderost und Reiskrankheiten, wie z.B. Pyricularia oryzae eingesetzt werden.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise 0,0001 bis 0,02 Gew.-%, am Wirkungsort erforderlich.

Herstellungsbeispiele

Beispiel 1

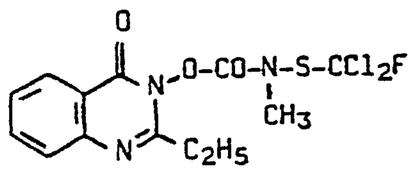

Eine Mischung aus 19 g (0,1 Mol) 2-Ethyl-3-hydroxy-3,4-dihydro-4-oxo-chinazolin, 20,6 g (0,15 Mol) Kaliumcarbonat, 200 ml Acetonitril und 21 g (0,1 Mol) N-Methyl-N-dichlorfluormethylsulfenyl-carbamoylfluorid wird 30 Minuten bei 40°C gerührt. Nach Abkühlen auf Raumtemperatur werden 300 ml Toluol zugegeben, dann schüttelt man zweimal mit je 300 ml Wasser aus. Die organische Phase wird über Natriumsulfat getrocknet und im Vakuum eingedampft. Zurück bleiben 29,6 g (78 % der Theorie) N-Methyl-N-dichlorfluormethylsulfenyl-O-(2-ethyl-3,4-dihydro-4-oxo-chinazolin-3-yl)-carbaminsäureester in Form farbloser Kristalle vom Schmelzpunkt 100°C.

Analog zu diesem Beispiel können die folgenden Verbindungen der Formel (I)

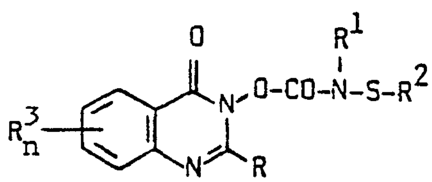

(I)

hergestellt werden:

| Beisp.-Nr. | R | $R^1$ | $R^2$ | $R^3$ | n | Schmelzpunkt °C |
|---|---|---|---|---|---|---|
| 2 | $CH_3$ | $CH_3$ | $CCl_2F$ | - | 0 | 106 |
| 3 | 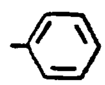 | $CH_3$ | $CCl_2F$ | - | 0 | 57 |
| 4 | $OCH_3$ | $CH_3$ | $CCl_2F$ | - | 0 | 90 |
| 5 | 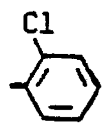 | $CH_3$ | $CCl_2F$ | - | 0 | 128 |
| 6 | 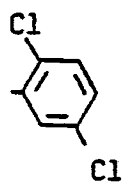 | $CH_3$ | $CCl_2F$ | - | 0 | 166 |

Herstellung der Ausgangsprodukte:

a)

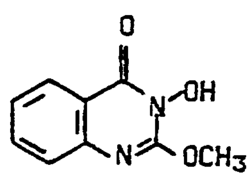

Eine Mischung aus 22,8 g (0,15 Mol) o-Amino-benzhydroxamsäure, 50 ml Methanol und 27,2 g (0,2 Mol) Orthokohlensäuretetramethylester wird 2 Stunden unter Rückfluß gekocht und dann auf 5°C abgekühlt. Das auskristallisierte Produkt wird abgesaugt. Man erhält so 17 g (60 % der Theorie) 2-Methoxy-3-hydroxy-3,4-dihydro-4-oxo-chinazolin in Form farbloser Kristalle mit dem Schmelzpunkt 220°C.

b)

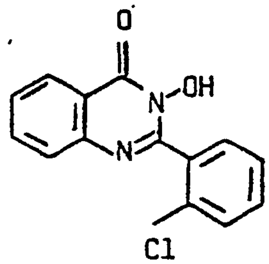

Zu einer Lösung von 30,4 g (0,2 Mol) o-Aminobenzhydroxamsäure in 250 ml Dioxan gibt man bei 20-30°C 35 g (0,2 Mol) o-Chlorbenzoylchlorid. Das Gemisch wird 3 Stunden unter Rückfluß gekocht, dann destilliert man das Lösungsmittel im Vakuum ab, gibt zum Rückstand 100 ml Ether und saugt das kristalline Produkt ab. Es wird in 300 ml 5-prozentiger Natronlauge gelöst, die Lösung wird filtriert und mit konz. Salzsäure angesäuert. Dann saugt man das ausgefallene Produkt ab und wäscht mit Wasser nach. Man erhält auf diese Weise 28,2 g (52 % der Theorie) 2-(2-Chlorphenyl)-3-hydroxy-3,4-dihydro-4-oxo-chinazolin in Form farbloser Kristalle vom Schmelzpunkt 211°C.

Analog können die folgenden Verbindungen der Formel (II)

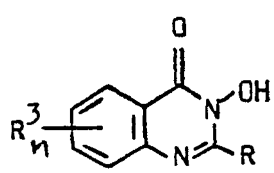

(II)

hergestellt werden:

| R | n | $R^3$ | Schmelzpunkt °C |
|---|---|---|---|
| c) $-OC_2H_5$ | 0 | - | 154 |
| d) $-CH_3$ | 0 | - | 215 |
| e) $-C_2H_5$ | 0 | - | 150 |
| f) -H | 0 | - | 244 |
| g) $-CH_2-S-CH_3$ | 0 | - | 171 |
| h) $-C_3H_7$-iso | 0 | - | 61 |
| i) $-C_3H_7$-n | 0 | - | 150 |
| j) 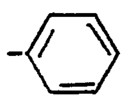 | 0 | - | 177 |
| k) 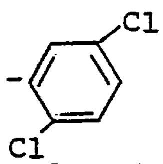 | 0 | - | 213 |

Anwendungsbeispiele

In den nachfolgenden Beispielen werden die hier angegebenen Verbindungen als Vergleichssubstanzen eingesetzt.

$$Cu(OH)_2 \cdot CuCl_2 \cdot H_2O \qquad (A)$$

basisches Kupferchlorid-Hydrat

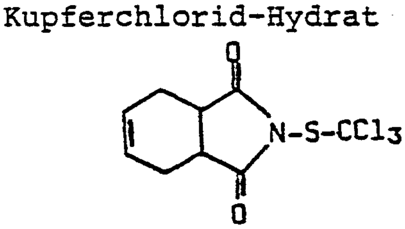

(B)

N-Trichlormethylthio-tetrahydrophthalimid

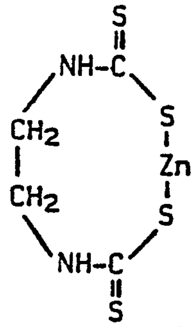

(C)

Zink-ethylen-1,2-bis-(dithiocarbamat)

Beispiel A

Xanthomonas oryzae-Test / Bakteriose / Reis
systemisch

Lösungsmittel: 121,25 Gewichtsteile Aceton
Emulgator: 3,75 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf systemische Eigenschaften werden 100 ml der Wirkstoffzubereitung auf Einheitserde gegossen, in der junge Pflanzen angezogen wurden. 3 Tage nach der Behandlung werden die Pflanzen mit einer wäßrigen Suspension von Xanthomonas oryzae durch Stichverletzung inokuliert. Danach verbleiben die Pflanzen 14 Tage bis zur Auswertung in einem Gewächshaus bei 24 bis 26°C und 70 bis 80 % rel. Luftfeuchtigkeit.

Eine deutliche Überlegenheit in der Wirksamkeit gegenüber dem Stand der Technik zeigt bei diesem Test z.B. die Verbindung gemäß Herstellungsbeispiel: 4.

Beispiel B

Botrytis-Test (Bohne) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Eine deutliche Überlegenheit in der Wirksamkeit gegenüber dem Stand der Technik zeigen in diesem Test z.B. die Verbindungen gemäß Herstellungsbeispiele: 1,2,3,4.

Beispiel C

Leptosphaeria nodorum-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 15°C und einer relativen Luftfeuchtigkeit von ca. 80% aufgestellt.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 1,2,3,4,5,6.

Patentansprüche

1. Oxochinazolin-carbaminsäureester der Formel

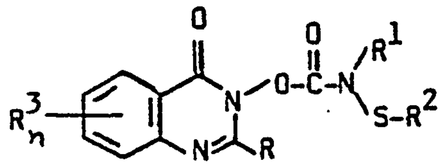

(I)

in welcher

R für Wasserstoff, Alkyl, Alkoxy, Alkoxyalkyl, Alkylthio, Alkylthioalkyl, Halogenalkyl oder gegebenenfalls substituiertes Aryl steht,

$R^1$ für Alkyl steht,

$R^2$ für Halogenalkyl steht,

$R^3$ für Alkyl oder Halogen steht und

n für eine Zahl von 0 bis 4 steht.

2. Oxochinazolin-carbaminsäureester gemäß Anspruch 1, wobei in der Formel I

R für Wasserstoff, für geradkettiges oder verzweigtes Alkyl, Alkoxy, Alkoxyalkyl, Alkylthio oder Alkylthioalkyl mit jeweils bis zu 6 Kohlenstoffatomen je Alkylrest, für Halogenalkyl mit bis zu 3 Kohlenstoffatomen und bis zu 7 gleichen oder verschiedenen Halogenatomen, oder für gegebenenfalls einfach oder mehrfach gleich oder verschieden substituiertes Aryl mit 6 bis 10 Kohlenstoffatomen steht, wobei als Substituenten in Frage kommen: Alkyl mit bis zu 4 Kohlenstoffatomen, Halogenalkyl mit bis zu 3 Kohlenstoffatomen und bis zu 5 Halogenatomen und Halogen,

$R^1$ für geradkettiges oder verzweigtes Alkyl mit bis zu 6 Kohlenstoffatomen steht,

$R^2$ für Halogenalkyl mit bis zu 3 Kohlenstoffatomen und bis zu 7 gleichen oder verschiedenen Halogenatomen steht,

$R^3$ für geradkettiges oder verzweigtes Alkyl mit bis zu 4 Kohlenstoffatomen oder für Halogen steht,

n für eine Zahl von 0 bis 3 steht.

3. Oxochinazolin-carbaminsäureester gemäß Anspruch 1, wobei in der Formel I

R für Wasserstoff, für geradkettiges oder verzweigtes Alkyl, Alkoxy, Alkoxyalkyl, Alkylthio oder Alkylthioalkyl mit jeweils 1 bis 4 Kohlenstoffatomen je Alkylrest, für Halogenalkyl mit 1 oder 2 Kohlenstoffatomen und 1 bis 5 gleichen oder verschiedenen Halogenatomen oder für gegebenenfalls ein- bis dreifach, gleich oder verschieden substituiertes Phenyl steht, wobei als Substituenten genannt seien: Fluor, Chlor, Brom, Methyl, Ethyl, Trifluormethyl und Trichlormethyl,

$R^1$ für geradkettiges oder verzweigtes Alkyl mit 1 bis 4 Kohlenstoffatomen steht,

$R^2$ für Halogenalkyl mit 1 oder 2 Kohlenstoffatomen und 1 bis 5 gleichen oder verschiedenen Halogenatomen steht,

$R^3$ für Methyl, Ethyl, Fluor, Chlor oder Brom steht und

n für die Zahl 0, 1 oder 2 steht.

4. Verfahren zur Herstellung von Oxochinazolin-carbaminsäureestern der Formel (I)

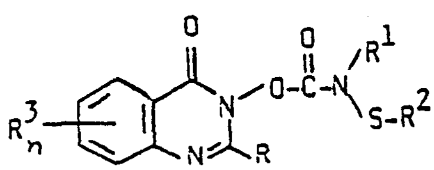

(I)

in welcher

R für Wasserstoff, Alkyl, Alkoxy, Alkoxyalkyl, Alkylthio, Alkylthioalkyl, Halogenalkyl oder gegebenenfalls substituiertes Aryl steht,

$R^1$ für Alkyl steht,

$R^2$ für Halogenalkyl steht und

$R^3$ für Alkyl oder Halogen steht und

n für eine Zahl von 0 bis 4 steht,

dadurch gekennzeichnet, daß man 3-Hydroxy-4-oxo-3,4-dihydrochinazoline der Formel (II)

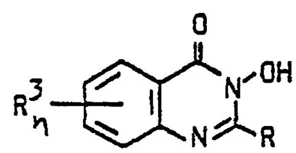

(II)

in welcher
R, $R^3$ und n die oben angegebene Bedeutung haben,

mit Carbamoylhalogeniden der Formel (III)

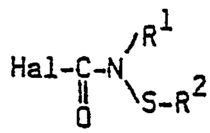

(III)

in welcher
$R^1$ und $R^2$ die oben angegebene Bedeutung haben und
Hal für Halogen steht,

in Gegenwart eines Säurebindemittels und gegebenenfalls in Gegenwart eines Verdünnungsmittels umsetzt.

5. Schädlingsbekämpfungsmittel, gekennzeichnet durch einen Gehalt an mindestens einem Oxochinazolin-carbaminsäureester der Formel (I).

6. Verwendung von Oxochinazolin-carbaminsäureestern der Formel (I) zur Bekämpfung von Schädlingen.

7. Verfahren zur Bekämpfung von Schädlingen, dadurch gekennzeichnet, daß man Oxochinazolin-carbaminsäureester der Formel (I) auf Schädlinge und/oder ihren Lebensraum einwirken läßt.

8. Verfahren zur Herstellung von Schädlingsbekämpfungsmitteln, dadurch gekennzeichnet, daß man Oxochinazolin-carbaminsäureester der Formel (I) mit Streckmitteln und/oder oberflächenaktiven Mitteln vermischt.

0101902

Europäisches Patentamt

# EUROPÄISCHER RECHERCHENBERICHT

Nummer der Anmeldung

EP 83 10 7180

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| A | GB-A-1 153 481 (FISONS PEST CONTROL)<br>* Seiten 1-5, 12-14 *<br>----- | 1,4,5-8 | C 07 D 239/92<br>C 07 D 239/96<br>A 01 N 47/18 |
| | | | RECHERCHIERTE SACHGEBIETE (Int. Cl. 3) |
| | | | C 07 D 239/00 |

Der vorliegende Recherchenbericht wurde für alle Patentansprüche erstellt.

| Recherchenort | Abschlußdatum der Recherche | Prüfer |
|---|---|---|
| DEN HAAG | 18-11-1983 | FRANCOIS J.C.L. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument